# EUROPEAN PATENT APPLICATION

(11) **EP 2 689 874 A1**
(43) Date of publication of application: **29.01.2014**
(21) Application number: 12762831.1
(22) Date of filing: 23.03.2012
(51) Int. Cl.: B22F 9/16, C09K 11/06, G01N 33/52, B82Y 40/00

(54) **METHOD FOR PRODUCING METALLIC NANOPARTICLES FUNCTIONALIZED WITH FLUORESCENT ORGANIC MOLECULES**

(30) Priority: 25.03.2011 ES 201130447
(71) Applicant: Universidad Pablo De Olavide, 41013 Sevilla (ES); Consejo Superior De Investigaciones Científicas (CSIC), 28006 Madrid (ES)
(72) Inventor: ZADERENKO PARTIDA, Ana Paula, E-41807 Espartinas (Sevilla) (ES); CARO SALAZAR, Carlos, E-41010 Sevilla (ES); MEJÍAS ROMERO, José Antonio, E-41089 Dos Hermanas (Sevilla) (ES); SAYAGUÉS DE VEGA, María Jesús, E-41092 Sevilla (ES)
(74) Representative: ABG Patentes, S.L.
(86) International application number: PCT/ES2012/070195
(87) International publication number: WO 2012/131133

(57) **Abstract**

The present invention relates to a method of obtaining metal nanoparticles functionalized with fluorescent organic molecules by means of the treatment of one or more metal salts with a reducing agent in the presence of a fluorescent organic molecule. The invention also relates to the metal nanoparticles obtainable by said method and to their use for detecting molecules and analytes.

## Description

### Field of the Art

The present invention relates to metal nanoparticles with fluorescent properties, to a method for preparing them and to their uses.

### State of the Art

Detection by means of fluorescence techniques forms the basis of most biological assays available today. Nevertheless, fluorescent detection at the molecular level has serious limitations resulting from the use of organic fluorophores, such as a low signal-to-noise ratio, low fluorophore photostability and high fluorophore photoblinking.

An enormous effort has been made in the last decade to find alternatives to the use of fluorescent organic molecules that offer an acceptable solution to the limitations of these molecules. In this regard, semiconductor nanoparticles or quantum dots (QD) have quantum efficiencies that are similar to those of organic fluorophores but higher extinction coefficients and higher photostability [Gao, X.; Yang, L.; Petros, J.A., Curr. Opin. Biotechnol. 2005, 16, 63-72]. Although QDs are very promising in biological applications [Fernandez-Arguelles, M.T.; Costa-Fernandez, J.M.; Pereiro, R.; Sanz-Medel, A., Analyst. 2008, 133, 444-447; Xue, X.; Pan, J.; Xie, H.; Wang, J.; Zhang, S., Talanta 2009, 77, 1808-1813; Ishihama, Y.; Funatsu, T., Biochem. Biophys. Res. Commun. 2009, 381, 33-38; Yong, K.T.; Roy, I.; Ding, H.; Bergey, E.J.; Prasad, P.N., Small. 2009, 5, 1997-2004; Bentolila, L.A.; Ebenstein, Y.; Weiss, S. J., Nucl. Med. 2009, 50, 493-496; Gill, R.; Zayats, M.; Willner, I., Angew. Chem. Int. Ed. Engl. 2008, 47, 7602-7625], they also have serious drawbacks due to their surface properties, such as the need to use organic solvents for synthesis, the complexity of their synthesis, their low stability in water, their difficult functionalization or the non-specific binding to biomolecules. In addition to their unfavorable surface properties, another problem with QDs for *in vivo* applications is their high toxicity [Drummen, P.C., Int. J. Mol. Sci. 2010, 11, 154-163; Singh, N.; Manshian, B.; Jenkins, G.J.; Griffiths, S.M.; Williams, P.M.; Maffeis, T.G.; Wright, C.J.; Doak, S.H., Biomaterials. 2009, 30, 3891-3914; Lewinski, N.; Colvin, V.; Drezek, R. Small. 2008, 4, 26-49; Hsieh, M.S.; Shiao, N.H.; Chan, W.H. Int. J. Mol. Sci. 2009, 10, 2122-2135; Roiter, Y.; Ornatska, M.; Rammohan, A.R.; Balakrishnan, J.; Heine, D.R.; Minko, S., Langmuir 2009, 25, 6287-6299; Chen, J.; Hessler, J.A.; Putchakayala, K.; Panama, B.K.; Khan, D.P.; Hong, S.; Mullen, D.G.; Dimaggio, S.C.; Som, A.; Tew, G.N.; Lopatin, A.N.; Baker, J.R.; Holl, M.M.; Orr, B.G., J. Phys. Chem. B. 2009, 113, 11179-11185]. In turn, such nanoparticles do not allow detection by means of surface-enhanced vibrational spectroscopies, their use being limited to detection by fluorescence.

It is known that metal nanoparticles interact with fluorophores close to their surface affecting their emission intensity, either reducing it or enhancing it. When this interaction leads to an increase in fluorescence, the effect is known as Metal Enhanced Fluorescence (MEF). MEF effect can be explained by the confluence of two mechanisms, on one hand, the increase in the incident electromagnetic fields associated with the metal nanoparticle leads to an increase in fluorophore emission, similarly to what occurs in the SERS (Surface-Enhanced Raman Spectroscopy) effect. On the other hand, the interaction of the fluorophore with the nanoparticle surface plasmon leads to an increase in both radioactive and non-radioactive decay rates and, accordingly, to the increase in quantum efficiency and the decrease in life time [Lakowicz, J.R.; Gryczynski, I.; Malicka, J.; Gryczynski, Z.; Geddes, C.D., J. Fluores. 2002, 12, 299-302; Lakowicz, J. R., Analytical Biochem. 2005, 337, 171-194; Cade, N. I.; Ritman-Meer, T.; Kwakwa K. A.; Richards, D., Nanotechnology 2009, 20, 285201-285206]. Additionally, and due to the decrease in life time, fluorophore photostability increases [Lakowicz, J.R.; Shen, Y.; D'Auria, S.; Malicka, J.; Fang, J.; Gryczynski, Z.; Gryczynski, I., Analytical Biochem. 2002, 301, 261-277; Lakowicz, J.R.; Malicka, J.; D'Auria, S.; Gryczynski, I., Analytical Biochem. 2003, 320, 13-20; Lukomska, J.; Malicka, J.; Gryczynski, I.; Lakowicz, J. R., J. Fluores. 2004, 14, 417-423]. Some of the factors that affect obtaining the MEF effect are: nature of the metal, nanoparticle-to-fluorophore distance, nanoparticle aggregation state, and spectral overlap between the nanoparticle surface plasmon and fluorophore emission [Huang, T.; Murray, R.W., Langmuir 2002, 18, 7077-7081; Kühn, S.; Håkanson, U.; Rogobete, L.; Sandoghdar, V., Phys. Rev. Lett. 2006, 97, 17402-17405; Anger, P.; Bharadwaj, P.; Novotny, L., Phys. Rev. Lett. 2006, 96, 113002-113003; Zhang, J.; Malicka, J.; Gryczynski, I.; Lakowicz, J. R., Analytical Biochem. 2004, 330, 81-86; Zhang, J.; Fu, Y.; Chowdhury, M. H.; Lakowicz, J. R., Nano Lett. 2007, 7, 2101-2107; Chen, Y.; Munechika, K.; Ginger, D. S., Nano Lett. 2007, 7. 690-696].

The distance between the fluorophore and the nanoparticle surface is critical. In this regard, it has been described that the fluorescence of a fluorophore is deactivated when the distance to the metal is too small (Anger, P.; Bharadwaj, P.; Novotny, L., Phys. Rev. Lett. 2006, 96, 113002-113003; Principles of nano-optics, L. Novotny, B. Hecht, Cambridge University Press, p. 346). The ideal nanoparticle-to-metal distance is in the range of 4-10 nm, making it necessary to use spacers in fluorophore-nanoparticle conjugation to obtain this distance, which makes it difficult to functionalize the nanoparticles with the fluorophore because synthesis in several steps and chemical modification of the fluorophore are required.

Therefore, there is a need to provide new fluorescent agents stable in aqueous medium that are biocompatible and can be easily functionalized with molecules and biomolecules of interest, and which can be used in detection assays using fluorescence techniques, as well as simpler methods that allow obtaining said fluorescent agents.

### Object of the Invention

The technical problem to be solved by the present invention is obtaining metal nanoparticles functionalized with fluorescent organic molecules, in which the fluorophore is not deactivated due to proximity to the metal, which are stable in aqueous medium, capable of being functionalized with other molecules of interest (for example, antibodies for biomedical applications) and biocompatible. Furthermore, these nanoparticles must preferably be able to be detected by techniques other than fluorescence, specifically UV-Vis, IR and Raman.

The present invention establishes a simple method of obtaining metal nanoparticles with fluorescent properties in a single step from solutions of metal salts, such as silver, gold, copper, aluminum, platinum, cobalt and palladium salts, by means of treatment in aqueous medium with a reducing agent in the presence of a fluorescent organic molecule. The invention also relates to the metal nanoparticles obtainable by said method and to their use as biomarkers.

Unlike common synthesis methods, in the present invention the fluorophore is added to the aqueous medium in which reduction is performed. As discussed above, since spacers are not used to bind the fluorophore to the nanoparticle, the fluorophore could be expected to become deactivated upon being absorbed directly on the nanoparticle surface. However, the inventors have surprisingly found that the addition of the fluorophore to the reduction medium leads to functionalized nanoparticles in which the fluorophore is not deactivated.

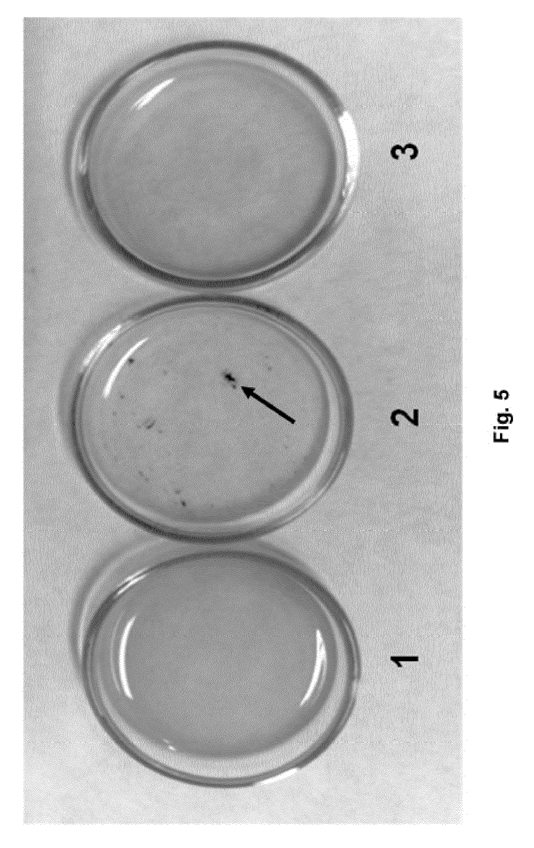

Additionally, the inventors also observed that the presence of the fluorescent organic molecule during the reduction process leads to stable nanoparticles. In contrast, if the fluorescent molecule is subsequently added on the already prepared metal nanoparticles, unstable, aggregating nanoparticles are obtained (Figure 5).

On the other hand, polymers which also bind to the nanoparticle surface can be incorporated. Said polymers can be added either to the reduction medium, together with the fluorophore, or they can subsequently be incorporated once the metal nanoparticles are formed. These polymers make the nanoparticles more stable in aqueous medium and provide them with a first organic layer with functional groups suitable for subsequent functionalization processes.

In addition to the simplicity of their synthesis and the functionalization possibilities that these nanoparticles offer, it must be pointed out that they have both the advantageous properties characteristic of metal nanoparticles (intense surface plasmon in the visible spectrum, capacity to be detected by means of surface-enhanced spectroscopies) and those of QDs (intense fluorescence).

### Detailed Description

The term "nanoparticle" is understood to be a structure with a mean particle size less than 1 µm, and generally comprised between 1 and 100 nm.

One aspect of the present invention is a new method of obtaining metal nanoparticles functionalized with fluorescent organic molecules which comprises treating one or more metal salts with a reducing agent capable of reducing the metal salt cation to the metallic state, in aqueous medium and in the presence of a fluorescent organic molecule.

In one embodiment of the present invention, the method of obtaining metal nanoparticles functionalized with fluorescent organic molecules comprises the following steps:
(a) Preparing an aqueous solution comprising one or more metal salts and a fluorescent organic molecule,
(b) Mixing the reducing agent capable of reducing the metal salt cation to the metallic state, optionally in the form of an aqueous solution comprising said reducing agent, with the solution of step (a),
(c) Optionally collecting the nanoparticles.

When the fluorescent organic molecule is not water-soluble, the molecule can previously be dissolved in a water-miscible organic solvent to form the solution of step (a). Therefore, in a particular embodiment the solution of step (a) can further comprise a water-miscible organic solvent, preferably an alcohol such as methanol, ethanol, propanol or butanol, or acetone, for example. According to one embodiment of the invention, when the fluorescent organic molecule is fluorescein the molecule is previously dissolved in ethanol before forming the solution of step (a).

In one embodiment of the present invention, the method is performed at a temperature between 0 and 100°C, preferably between 0 and 80°C, more preferably between 0 and 60°C, even more preferably between 0 and 40°C.

In another embodiment of the present invention, the method is performed at a temperature between 0 and 30°C, preferably between 0 and 10°C, more preferably between 0 and 4°C.

In a particular embodiment, the metal salts are selected from the group comprising silver, gold, copper, aluminum, platinum, cobalt and palladium salts, preferably from the group comprising gold or silver salts, more preferably AgNO₃, HAuCl₄ or their hydrates, such as HAuCl₄·3H₂O.

The reducing agents capable of reducing the metal salt cation to the metallic state useful for reducing metal salts are known by the person skilled in the art (Bradley, J. S., Schmid, G., Talapin, D. V., Shevchenko, E. V. and Weller, H. (2005) Syntheses and Characterizations: 3.2 Synthesis of Metal Nanoparticles, in Nanoparticles: From Theory to Application (ed G. Schmid), Wiley-VCH Verlag GmbH & Co. KGaA, Weinheim, FRG. doi: 10.1002/3527602399.ch3b. ISBN: 9783527305070). In a particular manner, hydrides, citrates, alcohols, reducing polymers, hydrazine, hydroxylamine or their derivatives and mixtures thereof can be used as reducing agents. In a particular embodiment, the reducing agent is selected from NaBH₄, sodium citrate, preferably NaBH₄, trisodium citrate, hydroxylamine, polyvinylpyrrolidone (PVP) and their mixtures.

The fluorescent organic molecules are those which are capable of emitting fluorescent light when they are illuminated with a radiation having the suitable wavelength. Examples of fluorescent organic molecules are known and the person skilled in the art can find them in reference documents such as, for example, "Fluorescent and Luminescent Probes for Biological Activity. A Practical Guide to Technology for Quantitative Real-Time Analysis", 2nd Ed. W. T. Mason, Academic Press (1999). In a particular embodiment, the fluorescent organic molecule is selected from fluorescein, rhodamines, coumarins, methylene blue, Cascade blue, methyl orange, Lucifer yellow, boron-dipyrromethane and mixtures thereof. The fluorescent organic molecule is preferably selected from rhodamine 6G, fluorescein and 3-hydroxycoumarin.

In another particular embodiment, the fluorescent organic molecule is a dye, such as for example methylene blue, Cascade blue, methyl orange, etc., metal nanoparticles having, besides surface plasmon emission, a strong emission due to the dye conjugated to the nanoparticle being obtained.

In one embodiment of the present invention, the concentration of the fluorescent molecule in the aqueous solution of step (a) defined above is in a range between 10⁻¹ and 10⁻⁶ M, preferably between 10⁻² and 10⁻⁵ M, more preferably between 10⁻² and 10⁻⁴ M, even more preferably between 0.8·10⁻³ M and 1.2·10⁻³ M.

Although the obtained nanoparticles are stable in aqueous medium, it is possible to even further enhance their stability by means of adding polymers. Furthermore, these polymers provide the nanoparticles with a first organic layer with functional groups suitable for subsequent functionalization processes. Therefore, according to a particular embodiment the metal nanoparticles obtained by means of the method of the invention are coated with a polymer. In a particular embodiment, obtaining the metal nanoparticles functionalized with a fluorescent organic molecule and the step of coating with a polymer are performed in a one-pot process, i.e., the polymer is added to the aqueous solution obtained after adding the reducing agent without previously isolating the nanoparticles obtained after the reduction step.

In one embodiment of the present invention, the method of obtaining metal nanoparticles functionalized with fluorescent organic molecules comprises the following steps:
(a) Preparing an aqueous solution comprising one or more metal salts and a fluorescent organic molecule,
(b) Mixing the reducing agent capable of reducing the metal salt cation to the metallic state, optionally in the form of an aqueous solution comprising said reducing agent, with the solution obtained in step (a),
(c) Adding a polymer, optionally dissolved in water, to the mixture resulting from step(b),
(d) Optionally collecting the nanoparticles.

According to another embodiment of the present invention, the polymer is part of the initial aqueous solution together with the one or more metal salts and the fluorescent organic molecule. Therefore, in one embodiment of the present invention the method of obtaining metal nanoparticles functionalized with fluorescent organic molecules comprises the following steps:
(a) Preparing an aqueous solution comprising one or more metal salts, a fluorescent organic molecule and a polymer,
(b) Mixing a reducing agent capable of reducing the metal salt cation to the metallic state, optionally in the form of an aqueous solution comprising said reducing agent, with the solution obtained in step (a),
(c) Optionally collecting the nanoparticles.

In this case, when the polymer is present in the solution during the reduction step, the resulting nanoparticles are even more stable than when the polymer is added on the already obtained nanoparticles after the reduction step.

In the present invention, the term "polymer" includes oligomers and both homopolymers and copolymers, and can be selected from natural and synthetic polymers. In a particular embodiment, the polymer used is a biocompatible polymer. The term biocompatible polymer refers to a polymer formed by a biocompatible material, i.e., it has no toxicity nor does it induce adverse reactions in biological systems. In one embodiment, the biocompatible polymer is selected from the group consisting of polysaccharides, such as dextran, pectin, carrageenan, chitosan or derivatives thereof; cyclodextrins; cellulose or cellulose derivatives; starch or starch derivatives; polyvinylpyrrolidone; polyethylene glycol; lactic and glycolic acid polymers as well as their copolymers; and mixtures thereof. Polyvinylpyrrolidone (PVP) and polyethylene glycol (PEG) are preferably used.

Preferably polymers with a mean molecular weight greater than 12,000 Da, more preferably greater than 20,000 Da and more preferably between 27,000 and 60,000 Da, are used as polyvinylpyrrolidone.

In another embodiment of the invention, the obtained nanoparticles are collected preferably by means of centrifugation and optionally purified. The nanoparticles can be purified by means of washing/centrifugation processes in a suitable solvent, preferably water, or by means of dialysis.

Another aspect of the present invention relates to metal nanoparticles functionalized with fluorescent organic molecules obtainable by means of any of the methods described above.

The nanoparticles of the present invention can be detected by fluorescence. In a particular embodiment, the nanoparticles can also be detected by UV-Vis, IR and/or Raman.

Another aspect of the invention relates to the use of metal nanoparticles functionalized with fluorescent organic molecules obtainable by means of the methods described above for detecting molecules or analytes, such as cells, viruses, bacteria, proteins, enzymes, peptides, amino acids, nucleic acids, nucleotides, carbohydrates, antibodies, antigens, drugs or the like. Detection is preferably performed by means of fluorescence techniques or surface-enhanced vibrational spectroscopies.

### Detailed Description of the Drawings

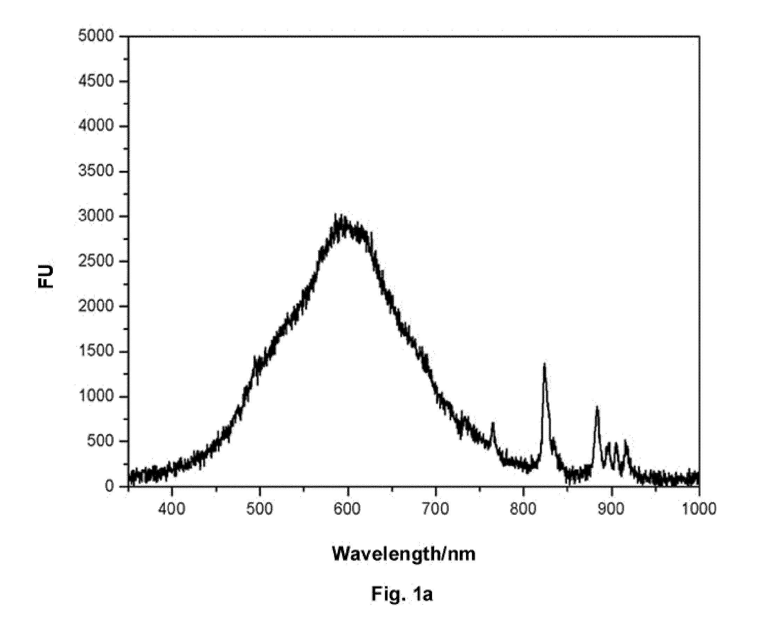

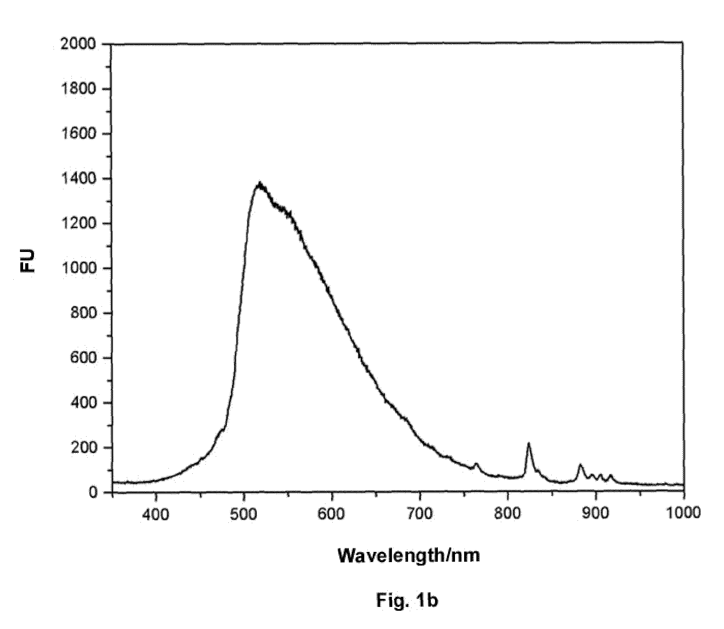

Figure 1 shows the fluorescence spectrum of silver nanoparticles functionalized with rhodamine 6G (a) and fluorescein (b), obtained according to Example 2, recorded in an Ocean Optics USB2000+ fluorometer.

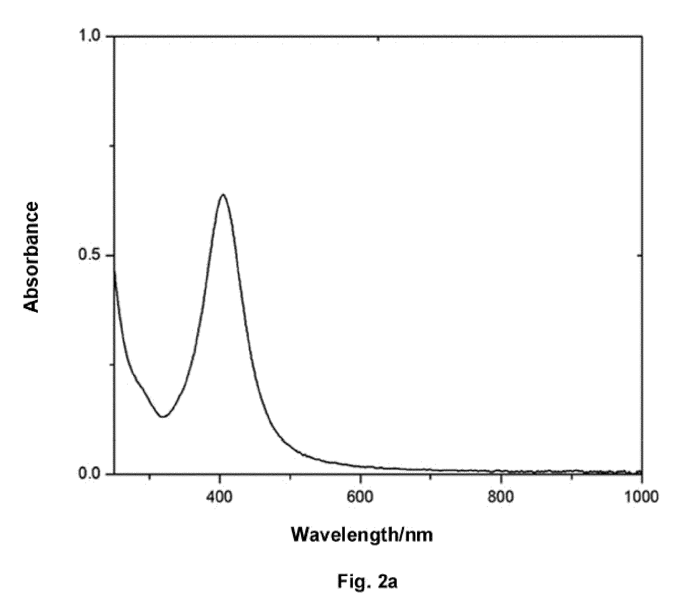

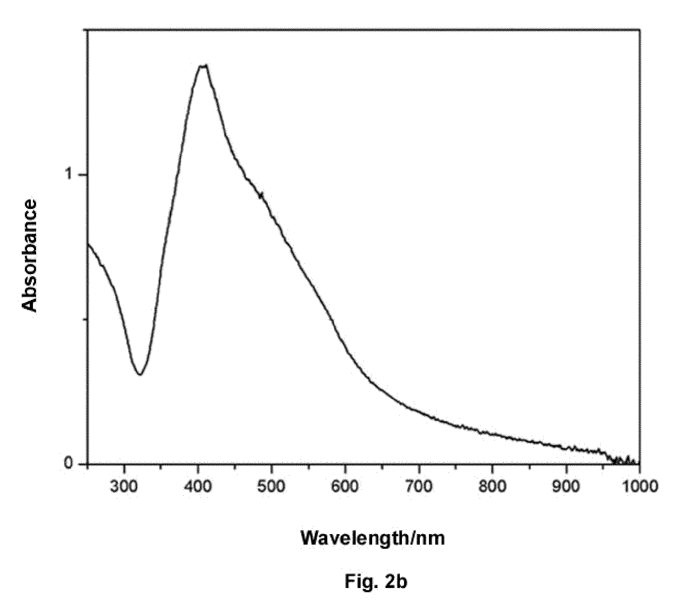

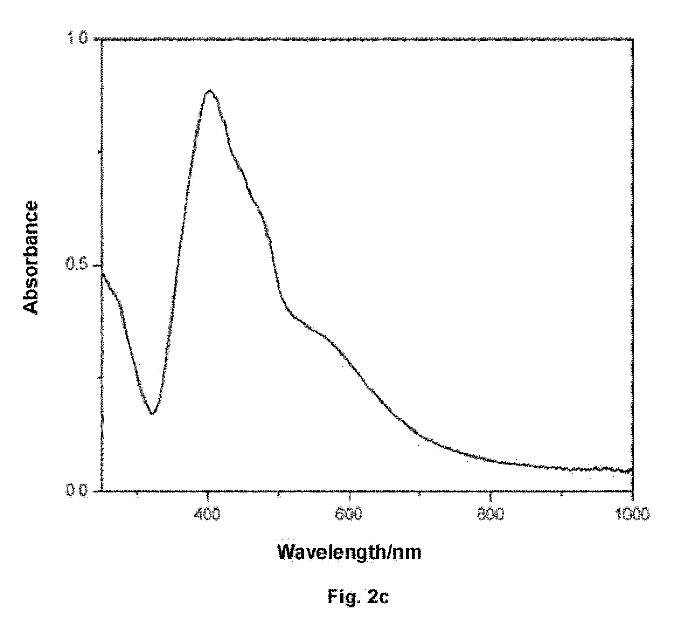

Figure 2 shows UV-Visible spectra, obtained with an Ocean Optics spectrometer equipped with an HR4000 detector, of the silver nanoparticles functionalized with PVP (a), silver nanoparticles functionalized with rhodamine 6G and PVP (b), obtained according to Example 2, and silver nanoparticles functionalized with fluorescein and PVP (c), obtained according to Example 1.

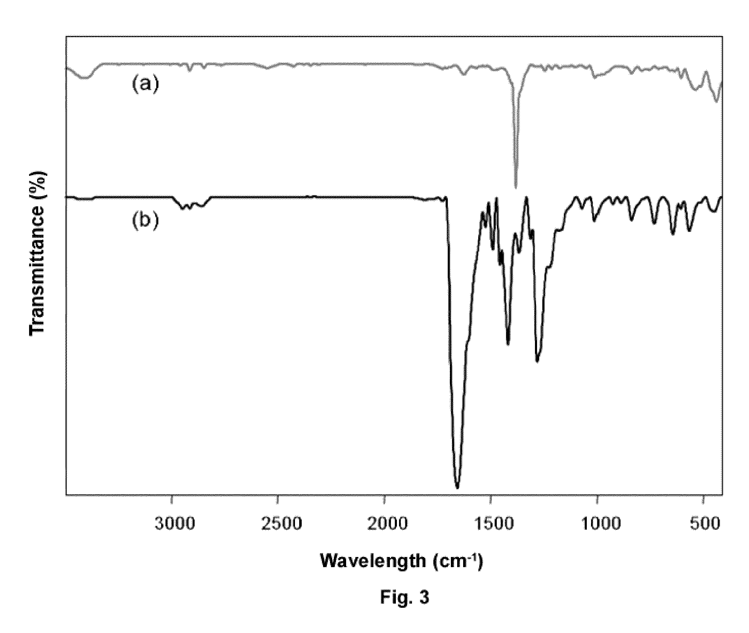

Figure 3 shows the IR spectrum of silver nanoparticles (a) obtained by silver nitrate reduction with NaBH₄ in aqueous medium, (b) silver nanoparticles obtained according to Example 2. The spectra were recorded in an FTIR Bruker IFS 66/s spectrometer with a resolution of 2 cm⁻¹ and 100 scans.

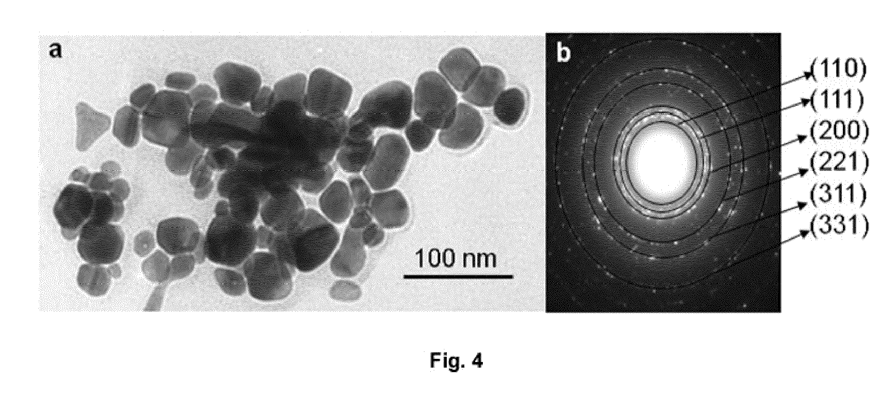

Figure 4 shows the microstructural characterization of the nanoparticles of Example 2. Figure 4(a) shows a transmission electron microscopy image and Figure 4(b) shows the corresponding electron diffraction pattern of the silver nanoparticles functionalized with rhodamine 6G and protected with PVP during synthesis (Example 2 of this specification). The nanometric nature of the nanoparticles, with a size comprised between 25 and 50 nm, and the faceted shape in most of them, clearly seeing the edges, can be seen in the micrograph. The layer of amorphous material of about 10 nm surrounding the silver nanoparticles is also seen. The nanocrystalline nature is seen in the electron diffraction pattern, all the diffraction rings have been assigned to planes (hkl) corresponding to metallic silver (marked in the figure). The TEM image was obtained in a Philips CM200 transmission electron microscope with a LaB₆ filament (point resolution of 0.3 nm) and acceleration voltage of 200 kV.
Figure 5 shows the comparison between the stability of the nanoparticles of the invention and the stability of nanoparticles to which the fluorescent organic molecule and the polymer are added after synthesis. 1: Silver nanoparticles synthesized by silver nitrate reduction with NaBH₄ in aqueous medium. 2: Silver nanoparticles synthesized by silver nitrate reduction with NaBH₄ in aqueous medium, after adding rhodamine 6G and PVP at the same concentrations as in Example 2 of the present application. The nanoparticles 2 are not stable, forming aggregates (the arrow in the figure points out one of these aggregates). 3: Nanoparticles obtained according to the method described in Example 2 of the specification.

### EXAMPLES

### Example 1. Synthesis of silver nanoparticles functionalized with rhodamine 6G and protected with PVP after synthesis

3.4 mg of silver nitrate (final concentration 1 mM) are dissolved in 20 mL of an aqueous solution of rhodamine 6G (10⁻³ M). 4 mL of an aqueous solution of NaBH₄ (10 mM) are added, dropwise and in an ice bath, to the resulting solution. Once the nanoparticles are obtained, 100 µL of an aqueous solution of PVP (1.4 mM; average molecular weight PVP: 29,000 g/mol) are added, and they are subjected to successive washing/centrifugation processes with Milli-Q water.

### Example 2. Synthesis of silver nanoparticles functionalized with rhodamine 6G and protected with PVP during synthesis

3.4 mg of silver nitrate (final concentration 1 mM) are dissolved in 20 mL of an aqueous solution of rhodamine 6G (10⁻³ M) and PVP (5% by weight). 4 mL of an aqueous solution of NaBH₄ (10 mM) are added, dropwise and in ice bath, to the resulting solution. Once the addition has ended, the obtained nanoparticles are purified by successive washing/centrifugation processes with Milli-Q water, followed by dialysis against Milli-Q water.

Figures 1 and 2 show the fluorescence and UV-Vis spectra, respectively, of the obtained nanoparticles, as well as the spectra of nanoparticles obtained according to Example 2, using fluorescein in the preparation instead of rhodamine 6G.

Figure 3 shows the IR spectrum of the nanoparticles obtained in embodiment 2 (Figure 3b), in comparison with the spectrum of the corresponding metal nanoparticles that are neither functionalized nor protected during the synthesis process (Figure 3a). As can be seen in the figure, the nanoparticles of the example have bands that are typical of rhodamine 6G and PVP.

Figure 4 shows the characterization of the nanoparticles by transmission electron microscopy.

As shown in Figure 5, the nanoparticles obtained by this method are stable (3). However, the nanoparticles obtained by means of a similar method, but adding rhodamine 6G and PVP after forming the metal nanoparticles, are not stable, which leads to the occurrence of large aggregates (2). Aggregate formation is also clearly shown in the disappearance of the nanoparticle plasmon band in the UV-Visible spectrum. In contrast, neither nanoparticles 1 nor nanoparticles 3 aggregate, showing surface plasmon bands in the corresponding UV-Visible spectra.

### Example 3. Synthesis of silver nanoparticles functionalized with rhodamine 6G and protected with PVP during synthesis, using PVP as a reducing agent

10 mL of an aqueous solution of rhodamine 6G (10⁻³ M) and silver nitrate (1 mM) are added to 10 mL of an aqueous solution of rhodamine 6G (10⁻³ M) and PVP (10% by weight), previously heated to 65°C. The mixture is left to react for 6 hours at 65°C and, after this time, the obtained nanoparticles are collected and purified by means of successive washing/centrifugation processes with Milli-Q water, followed by dialysis against Milli-Q water.

## Claims

1. A method for obtaining metal nanoparticles functionalized with fluorescent organic molecules which comprises treating one or more metal salts with a reducing agent capable of reducing the metal salt cation to the metallic state in aqueous medium, **characterized in that** the treatment of the one or more metal salts with the reducing agent is performed in the presence of a fluorescent organic molecule.

2. The method according to claim 1, **characterized in that** it comprises the following steps:
(a)Preparing an aqueous solution comprising one or more metal salts and a fluorescent organic molecule,
(b)Mixing the reducing agent capable of reducing the metal salt cation to the metallic state, optionally in the form of an aqueous solution comprising said reducing agent, with the solution obtained in step (a), and
(c)Optionally collecting the nanoparticles.

3. The method according to any of the preceding claims, **characterized in that** the metal salts are selected from the group comprising silver, gold, copper, aluminum, platinum, cobalt and palladium salts.

4. The method according to any of the preceding claims, **characterized in that** the reducing agent is a hydride, citrate, alcohol, reducing polymer, hydrazine, hydroxylamine or mixtures thereof.

5. The method according to any of the preceding claims, **characterized in that** the fluorescent organic molecule is selected from fluorescein, rhodamine, coumarin, methylene blue, Cascade blue, Lucifer yellow, boron-dipyrromethane, and mixtures thereof

6. The method according to any of the preceding claims, **characterized in that** it is performed at a temperature between 0 and 100°C.

7. The method according to claim 6, **characterized in that** it is performed at a temperature between 0 and 30°C.

8. The method according to any of the preceding claims, **characterized in that** the reduction is performed in the presence of a polymer.

9. The method according to any of claims 2 to 8 **characterized in that** the aqueous solution defined in step (a) comprises a polymer.

10. The method according to any of the preceding claims, **characterized in that** the obtained nanoparticles are subsequently coated with a polymer.

11. The method according to any of claims 9 to 10, **characterized in that** the polymer is a biocompatible polymer.

12. The method according to claim 11, **characterized in that** the biocompatible polymer is selected from polyvinylpyrrolidone and polyethylene glycol.

13. Metal nanoparticles functionalized with fluorescent organic molecules obtainable by means of the method defined in any of claims 1 to 12.

14. Use of the nanoparticles of claim 13 for detecting molecules or analytes by means of fluorescence techniques or surface-enhanced vibrational spectroscopies.
